# EUROPEAN PATENT APPLICATION

(11) **EP 1 997 802 A1**
(43) Date of publication of application: **03.12.2008**
(21) Application number: 07738656.3
(22) Date of filing: 15.03.2007
(51) Int. Cl.: C07C 275/70, C07C 273/18, C07B 61/00

(54) **METHOD FOR NITRATING ISOUREA**

(30) Priority: 16.03.2006 JP 2006072577
(71) Applicant: Sumitomo Chemical Company, Limited, Tokyo 104-8260 (JP)
(72) Inventor: YAMAGATA, Kazuyuki, Oita-shi, Oita 8700025 (JP); SEKO, Shinzo, Toyonaka-shi, Osaka 5600003 (JP)
(74) Representative: Vossius & Partner
(86) International application number: PCT/JP2007/055206
(87) International publication number: WO 2007/105793

(57) **Abstract**

An object of the present invention is to industrially advantageously produce N-nitroisoureas or a salt thereof which is useful as a synthetic intermediate for pharmaceuticals and pesticides. The present invention relates to a process for producing a compound represented by the formula (2): 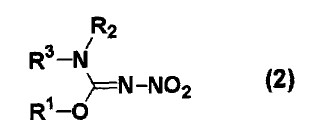 wherein R¹, R² and R³ are as defined below, or a salt thereof, which comprises reacting a compound represented by the formula (1): 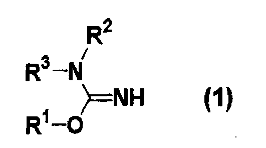 wherein R¹ represents an optionally substituted straight-chain or branched C₁ to C₆ alkyl group, R² and R³ are the same or different and represent an optionally substituted straight-chain or branched C₁ to C₆ alkyl group, a cycloalkyl group or a substituted aryl group, or R² and R³ simultaneously represent a hydrogen atom, or a salt thereof with a nitrating agent in the presence of sulfur trioxide.

## Description

### Technical Field

This application claims priority on Japanese Patent Application No. 2006-72577, the entire content of which is incorporated by reference herein.
The present invention relates to a process for nitrating isoureas.

### Background Art

It is known that N-nitroisoureas or a salt thereof is useful as a synthetic intermediate for pharmaceuticals and pesticides (Japanese Unexamined Patent Publication (Kokai) No. 9-67342). There is also known a process for reacting O-methylisourea or a salt thereof with fuming nitric acid (specific gravity: 1.5) in 98% sulfuric acid thereby nitrating the O-methylisourea or a salt thereof to produce an O-methyl-N-nitroisourea or a salt thereof (Japanese Unexamined Patent Publication (Kokai) No. 9-67342; Japanese Unexamined Patent Publication (Kokai) No. 2000-103775; and Recueil des Travaux Chimiques des Pays-Bas, Vol. 81, pp.69 (1962)).

Patent document 1: Japanese Unexamined Patent Publication (Kokai) No. 9-67342
Patent document 2: Japanese Unexamined Patent Publication (Kokai) No. 2000-103775
Non-patent document 1: Recueil des Travaux Chimiques des Pays-Bas, Vol. 81, pp.69 (1962)

### Disclosure of the Invention

An object of the present invention is to industrially advantageously produce N-nitroisourea.

The present invention relates to a process for producing a compound represented by the formula (2): 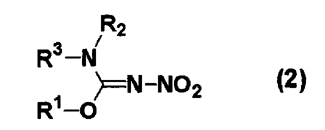 wherein R¹, R² and R³ are as defined below, or a salt thereof, which comprises reacting a compound represented by the formula (1): 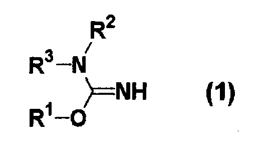 wherein R¹ represents an optionally substituted straight-chain or branched C₁ to C₆ alkyl group, R² and R³ are the same or different and represent an optionally substituted straight-chain or branched C₁ to C₆ alkyl group, a cycloalkyl group or a substituted aryl group, or R² and R³ simultaneously represent a hydrogen atom, or a salt thereof with a nitrating agent in the presence of sulfur trioxide.

According to the production process of the present invention, it is possible to reduce a waste acid derived from sulfuric acid used in the production of N-nitroisoureas or a salt thereof which is useful as a synthetic intermediate for pharmaceuticals and pesticides, or to improve a yield, and thus the present invention is industrially advantageous.

### Best Mode for Carrying Out the Invention

Substituents of the above-described formulas (1) and (2) in the present invention will be described below.
Examples of the optionally substituted straight-chain or branched C₁ to C₆ alkyl group represented by R¹, R² and R³ include, for example, a methyl group, an ethyl group, a propyl group, an isopropyl group, a butyl group, an isobutyl group, a sec-butyl group, a tert-butyl group, a pentyl group, and a hexyl group. Examples of the cycloalkyl group represented by R² or R³ include, for example, C₅-C₆ cycloalkyl groups such as a cyclopentyl group, a cyclohexyl group, or the like. Examples of the substituted aryl group include, for example, a phenyl group substituted with at least one group selected from the group consisting of a halogen atom and a nitro group, and specific examples thereof include a chlorophenyl group, a dichlorophenyl group, a nitrophenyl group, and the like.

Examples of the compound of the formula (1) include O-methylisourea, N,N,O-trimethylisourea, N,N,O-tripropylisourea, N,N,O-tributylisourea, N,N-dimethyl-O-ethylisourea, N,N-dibutyl-O-methylisourea, N,N,O-tributylisourea, N,N-dicyclohexyl-O-methylisourea, and O-butyl-N-(3,4-dichlorophenyl)isourea. The compound of the formula (1) can be produced, for example, according to the methods described in Japanese Unexamined Patent Publication (Kokai) No. 3-157358, Journal of Organic Chemistry, Vol. 26, pp.412 (1961), and Journal of Organic Chemistry, Vol. 28, pp.2653 (1963). The salts of the compounds of the above-described formulas (1) and (2) may be salts which are acceptable as an intermediate for pharmaceuticals or pesticides, and examples thereof include salts formed by using inorganic acids such as hydrochloric acid, sulfuric acid or the like, or salts formed by using organic acids such as picric acid, methanesulfonic acid, p-toluenesulfonic acid, or the like. Among these salts, hydrochlorides and sulfates are preferred. In the case of the compound represented by the formula (1), sulfate (R¹O-C(NR²R³)=NH·H₂S0₄) and 1/2 sulfate (R¹OC(NR²R³)=NH· l/2H₂SO₄) are particularly preferred.

The production process of the present invention can be carried out, for example, according to the following reaction conditions. When the product is obtained as a free form compound by the production process described below, the product can be converted into the above-described salts according to a conventional process. When the product is obtained as a salt by the process described below, the product can be converted into the isolated compound according to a conventional process.

In the present invention, it is preferred that nitric acid is used as the nitrating agent and the reaction is performed in the presence of sulfuric acid. Sixty to 100% nitric acid is usually used as the nitrating agent. In addition, an alkali metal nitrate such as sodium nitrate or potassium nitrate may be used in sulfuric acid as the nitrating agent, and also nitronium trifluoromethanesulfonate (NO₂CF₃SO₃) may be used.

In the present invention, the nitrating agent is usually used in an amount of 1 mol or more, preferably 1.5 or more, and more preferably 2.5 mol or more, per mol of the compound represented by the formula (1) or a salt thereof. The upper limit of the amount of the nitrating agent is not particularly limited and is usually up to 10 mol, and preferably up to about 3 mol, taking account of economical efficiency. It is preferred to use, as the nitrating agent, 90% by weight or more, particularly 97% by weight of more nitric acid so as to perform the nitration reaction in good yield.

The above-described reaction in the present invention is usually performed in the presence of a nitrating solvent. Examples of the nitrating solvent include, for example, acidic solvents such as sulfuric acid, acetic acid, acetic anhydride, trifluoroacetic anhydride, trifluoromethanesulfonic acid or the like. If necessary, a solvent or a mixture thereof, which does not exert an adverse influence on the reaction, may be used. Examples of the solvent include, in addition to the above-described acidic solvents, aromatic hydrocarbons such as chlorobenzene, o-dichlorobenzene, nitrobenzene or the like; halogenated hydrocarbons such as dichloromethane, chloroform, 1,2-dichloroethane, carbon tetrachloride or the like; saturated hydrocarbons such as hexane, heptane, cyclohexane or the like; and ethers such as diethyl ether, tetrahydrofuran, dioxane or the like. These solvents may be used alone, or two or more kinds of them may be optionally used by mixing in a proper mixing ratio, for example, about 1:1 to 1:10 (volume ratio).

In the present invention, particularly preferred nitrating solvent is sulfuric acid and is preferably used in an amount within a range from 2 to 3 mol per mol of the compound represented by the formula (1) or a salt thereof. It is preferred to use, as sulfuric acid, about 96% or more, more preferably about 98% or more of concentrated sulfuric acid. The amount of sulfuric acid is preferably 1.5 mol or more, more preferably 1.8 mol or more, still more preferably 2 mol or more, further preferably 2.3 mol or more, and particularly preferably 2.5 mol or more, per mol of the compound represented by the formula (1) or a salt thereof so as to perform the nitration reaction in good yield. The upper limit of the amount is not particularly limited, and is preferably up to about 3 mol so as to produce the compound of the formula(2) or a salt thereof in a good yield while suppressing cost and formation of a waste acid in an after-treatment of the reaction.

The nitration reaction in the present invention is usually performed by mixing the compound of the formula (1) or a salt thereof with the nitrating solvent in the presence of sulfur trioxide. When the nitrating solvent and the nitrating agent contain moisture, sulfur trioxide may be added taking account of the amount so that an effective amount of sulfur trioxide exists in the reaction system for nitration. The effective amount of sulfur trioxide to be added is preferably 0.1 mol or more, and more preferably 0.2 mol or more, per mol of the compound represented by the formula (1) or a salt thereof. The upper limit of the amount is not particularly limited as long as an adverse influence is not exerted on operability and economical efficiency of the reaction, and is preferably about 0.7 mol so as to reduce the amount of the waste acid.

In the case where the reaction is performed using sulfuric acid as the solvent of the nitration reaction, when sulfur trioxide is used in an amount of 0.24 mol or more per mol of the compound represented by the formula (1) or a salt thereof, an objective product can be obtained in good nitration yield even if the lower limit of the amount of sulfuric acid is adjusted to about 1.8 mol per mol of the compound represented by the formula (1) or a salt thereof, and thus the amount of the waste acid produced during the after-treatment of the reaction can be reduced.

When sulfuric acid is used as the solvent for the nitration reaction, sulfur trioxide can be used as fuming sulfuric acid prepared by preliminarily mixing sulfuric acid with sulfur trioxide. It is possible to employ a process in which a predetermined amount of sulfur trioxide generated by heating fuming sulfuric acid is introduced in a reaction vessel and mixed so as to realize the presence of sulfur trioxide.

The reaction temperature of the reaction in the present invention is usually from about -20 to 30°C, preferably from 0 to 30°C, and more preferably from about 10 to 25°C. The reaction time is usually from about 10 minutes to 20 hours, and preferably from about 30 minutes to 8 hours.

After completion of the reaction, the reaction mixture is diluted with water and/or ice to obtain a mixture containing the compound represented by the formula (2) or a salt thereof. Specifically, after completion of the reaction, the reaction mixture is poured into cold water, water, ice, or an optional mixture thereof to obtain a mixture containing the compound represented by the formula (2) or a salt thereof. When sulfuric acid is used as the solvent, the reaction mixture is diluted while paying attention to heat generation. In this case, the reaction temperature is usually within a range from -20 to 60°C, and preferably from -10 to 30°C. Alternatively, after completion of the reaction, in the case where a salt of a product (2) is precipitated by cooling the reaction mixture with ice water, the salt may be isolated by filtration.

The mixture containing the compound of the formula (2) or a salt thereof thus obtained can be isolated and purified by known means, for example, crystallization, filtration, recrystallization, solvent extraction, concentration, vacuum concentration, or chromatography.
The compound of the formula (2) or a salt thereof is capable of forming a cis-stereoisomer and a trans-stereoisomer according to the position of the nitro group. The compound of the formula (2) or a salt thereof is capable of theoretically forming a tautomer according to the substituent. The compound of the formula (2) or a salt thereof of the present invention also include all isomers or salts thereof or mixtures thereof. Examples of the compound of the formula (2) or a salt thereof thus obtained include O-methyl-N-nitroisourea, N,N,O-trimethyl-N'-nitroisourea, N,N,O-tripropyl-N'-nitroisourea, N,N-dimethyl-O-ethyl-N'-nitroisourea, N,N-dibutyl-O-methyl-N'-nitroisourea, N,N,O-tributyl-N'-nitroisourea, N,N-dicyclohexyl-O-methyl-N'-nitroisourea, O-butyl-N- (3,4-dichlorophenyl)-N'-nitroisourea, and salts of these compounds.

Main and preferred aspects of the present invention are listed below.
[1] A process for producing a compound represented by the formula (2): 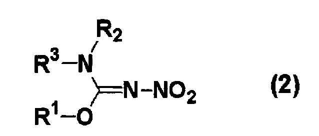 wherein R¹, R² and R³ are as defined below, or a salt thereof, which comprises reacting a compound represented by the formula (1): 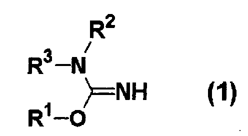 wherein R¹ represents an optionally substituted straight-chain or branched C₁ to C₆ alkyl group, R² and R³ are the same or different and represent an optionally substituted straight-chain or branched C₁ to C₆ alkyl group, a cycloalkyl group or a substituted aryl group, or R² and R³ simultaneously represent a hydrogen atom, or a salt thereof with a nitrating agent in the presence of sulfur trioxide.
[2] The process according to the above-described [1], wherein the nitrating agent is nitric acid and the reaction is performed in the presence of sulfuric acid.
[3] The process according to above-described [1] or [2], wherein the compound represented by the formula(1) is O-methylisourea sulfate or O-methylisourea 1/2 sulfate.
[4] The process according to any one of above-described [1] to [3], wherein sulfur trioxide is added in an amount within a range from 0.2 to 0.7 mol per mol of the compound represented by the formula (1) or a salt thereof.
[5] The process according to any one of above-described [2] to [4], wherein sulfuric acid is added in an amount within a range from 2 to 3 mol per mol of the compound represented by the formula (1) or a salt thereof.
[6] The process according to any one of above-described [2] to [5], wherein nitric acid is added in an amount within a range from 1.5 to 3 mol per mol of the compound represented by the formula (1) or a salt thereof.
[7] The process according to any one of above-described [1] to [6], wherein the reaction temperature is within a range from 10 to 25°C.

### Examples

The present invention will be described in detail below by way of Examples. The present invention disclosed above should not be limited to the following Examples as long as they may be included within the spirit and scope of the invention. Persons skilled in the art can easily employ known modifications and conditions based on the following descriptions.

### [Example 1]

In a mixture prepared by dissolving 27.9 g (content: 98%, 0.22 mol) of O-methylisourea 1/2 sulfate in 42.5 g (sulfuric acid: 0.39 mol; 1.76 equivalents, sulfur trioxide: 0.053 mol; 0.24 equivalents) of fuming sulfuric acid (sulfur trioxide concentration: 10 wt%), 34.9 g (0.54 mol; 2.5 equivalents) of 98% nitric acid was added dropwise at 10°C for one hour. After stirring for 6 hours, 103.2 g of a reaction solution was obtained. As a result of analysis by high performance liquid chromatography, the content of O-methyl-N-nitroisourea in the reaction solution was 24.1 wt%. A reaction yield was 94.1%.

### [Example 2]

In a mixture prepared by dissolving 62.0 g (content: 98%, 0.49 mol) of O-methylisourea 1/2 sulfate in 94.7 g (sulfuric acid: 0.87 mol; 1.8 equivalents, sulfur trioxide: 0.12 mol; 0.24 equivalents) of fuming sulfuric acid (sulfur trioxide concentration: 10 wt%), 77.8 g (1.21 mol; 2.5 equivalents) of 98% nitric acid was added dropwise at 10°C over one hour. After stirring for 6 hours, 234.1g of a reaction solution was obtained. As a result of analysis by high performance liquid chromatography, the content of O-methyl-N-nitroisourea in the reaction solution was 24.1 wt%. A reaction yield was 96.0%.

The resulting reaction solution was added dropwise in 194 g of water at 10°C or lower while stirring and then 470.5 g of a 27% sodium hydroxide solution was added at 20°C or lower thereby adjusting the pH of the mixture to a value of 7 to 8 through neutralization. To the neutralized mixture, 207.7 g of an aqueous 5-(aminomethyl)-2-chlorothiazole hydrochloride solution (content: 35.6 wt%, 0.40 mol) was added and the pH was adjusted to a value of 6.5 to 7.0 with 5% sodium hydroxide solution. The reaction solution was maintained at 20°C for 14 hours and maintained at 30°C for 12 hours after heating. During maintaining, the pH of the reaction solution was maintained at a value of 6.5 to 7.0 with 5% sodium hydroxide solution. The reaction solution was filtered and washed with warm water, and then the resultant wet cake was dried under reduced pressure to obtain 70.7 g of a white dry cake. As a result of analysis by high performance liquid chromatography, the content of N-(2-chlorothiazol-5-ylmethyl)-O-methyl-N'-nitroisourea was 97.2%. A reaction yield from 5-(aminomethyl)-2-chlorothiazole hydrochloride was 68.6%.

### [Example 3]

In a mixture prepared by dissolving 20.0 g (content: 98%, 0.16 mol) of O-methylisourea 1/2 sulfate in 38.6 g (sulfuric acid: 0.37 mol; 2.3 equivalents, sulfur trioxide: 0.024 mol; 0.15 equivalents) of fuming sulfuric acid (sulfur trioxide concentration: 5.0 wt%), 15.4 g (0.24 mol; 1.5 equivalents) of 98% nitric acid was added dropwise at 25°C over one hour. After stirring for 6 hours, 73.3 g of a reaction solution was obtained. As a result of analysis by high performance liquid chromatography, the content of O-methyl-N-nitroisourea in the reaction solution was 23.7 wt%. A reaction yield was 91.6%.

### [Example 4]

In a mixture prepared by dissolving 20.1 g (content: 98%, 0.16 mol) of O-methylisourea 1/2 sulfate in 46.6 g (sulfuric acid: 0.46 mol; 2.9 equivalents, sulfur trioxide: 0.016 mol; 0.1 equivalent) of fuming sulfuric acid (sulfur trioxide concentration: 2.8 wt%), 15.4 g (0.24 mol; 1.5 equivalents) of 98% nitric acid was added dropwise at 10°C over one hour. After stirring for 6 hours, 81.6 g of a reaction solution was obtained. As a result of analysis by high performance liquid chromatography, the content of O-methyl-N-nitroisourea in the reaction solution was 23.1 wt%. A reaction yield was 98.9%.

### [Comparative Example 1]

In a mixture prepared by dissolving 20.0 g (content: 98%, 0.16 mol) of O-methylisourea 1/2 sulfate in 47.8 g (0.48 mol; 3 equivalents) of 98% sulfuric acid, 15.3 g (0.24 mol; 1.5 equivalents) of 98% nitric acid was added dropwise at 10°C over one hour. After stirring for 6 hours, 82.7 g of a reaction solution was obtained. As a result of analysis by high performance liquid chromatography, the content of O-methyl-N-nitroisourea in the reaction solution was 20.8 wt%. A reaction yield was 90.7%.

The results are shown in Table 1.

**Table 1**

| | Amount of sulfuric acid | Amount of sulfur trioxide added | Amount of nitric acid | Reaction temperature/hours | Nitration reaction yield |
|---|---|---|---|---|---|
| Example 1 | 1.8 equivalents | 0.24 equivalents | 2.5 equivalents | 10°C/6hr | 94.1% |
| Example 2 | 1.8 equivalents | 0.24 equivalents | 2.5 equivalents | 10°C/6hr | 96.0% |
| Example 3 | 2.3 equivalents | 0.15 equivalents | 1.5 equivalents | 25°C/6hr | 91.6% |
| Example 4 | 2.9 equivalents | 0.1 equivalents | 1.5 equivalents | 10°C/6hr | 98.9% |
| Comparative Example 1 | 3 equivalents | None | 1.5 equivalents | 10°C/6hr | 90.7% |

## Claims

1. A process for producing a compound represented by the formula (2): 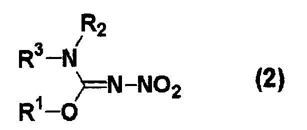 wherein R¹, R² and R³ are as defined below, or a salt thereof, which comprises reacting a compound represented by the formula (1): 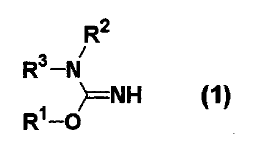 wherein R¹ represents an optionally substituted straight-chain or branched C₁ to C₆ alkyl group, R² and R³ are the same or different and represent an optionally substituted straight-chain or branched C₁ to C₆ alkyl group, a cycloalkyl group or a substituted aryl group, or R² and R³ simultaneously represent a hydrogen atom, or a salt thereof with a nitrating agent in the presence of sulfur trioxide.

2. The process according to claim 1, wherein the nitrating agent is nitric acid and the reaction is performed in the presence of sulfuric acid.

3. The process according to claim 1 or 2, wherein the compound represented by the formula (1) is O-methylisourea sulfate or O-methylisourea 1/2 sulfate.

4. The process according to any one of claims 1 to 3, wherein sulfur trioxide is added in an amount within a range from 0.2 to 0.7 mol per mol of the compound represented by the formula (1) or a salt thereof.

5. The process according to any one of claims 2 to 4, wherein sulfuric acid is added in an amount within a range from 2 to 3 mol per mol of the compound represented by the formula (1) or a salt thereof.

6. The process according to any one of claims 2 to 5, wherein nitric acid is added in an amount within a range from 1.5 to 3 mol per mol of the compound represented by the formula (1) or a salt thereof.

7. The process according to any one of claims 1 to 6, wherein the reaction temperature is within a range from 10 to 25°C.
